# EUROPEAN PATENT APPLICATION

(11) **EP 1 096 024 A1**
(43) Date of publication of application: **02.05.2001**
(21) Application number: 99870226.0
(22) Date of filing: 28.10.1999
(51) Int. Cl.: C12Q 1/68

(54) **Method and kit for the screening and/or the quantification of multiple homologous nucleic acid sequences on arrays**

(71) Applicant: Remacle, José, 5020 Malonne (BE)
(72) Inventor: Remacle, José, 5020 Malonne (BE); Zammatteo, Nathalie, 5100 Jambes (BE); De Longueville, Françoise, 5100 Jambes (BE); Alexandre, Isabelle, 5170 Lesve (BE); Hamels, Sandrine, 6280 Loverval (BE)
(74) Representative: Van Malderen, Joelle

(57) **Abstract**

The present invention is related to a new detection and/or quantification method of multiple target nucleotide sequences, possibly homologous to each other(s), and being present in the same biological sample, comprising the steps of:
- amplifying or copying at least a part of original nucleotide sequences present in the biological sample into target nucleotide sequences;
- putting into contact the obtained target nucleotide sequences with corresponding capture nucleotide sequences bounded to an insoluble solid support, characterised in that said capture nucleotide sequences have a (single stranded) length comprised between about 40 and about 400 base pairs, and in that said capture nucleotide sequences are bounded to the insoluble support according to an array with a density of at least 5 different capture nucleotide sequences / cm² surface of solid support, and
- detecting and/or quantifying a signal resulting from the formation of double stranded nucleotide sequences resulting from the hybridisation by complementary base pairing.

## Description

### Field of the invention

The present invention is related to a method and kit comprising reagents for simultaneous detection and/or quantification of multiple homologous nucleic acid sequences on arrays.

### Background of the invention

The development of miniaturisation, especially the array technology, is particularly useful for simultaneous detection of a great number of nucleic acids sequences present in biological samples. The array contains on its surface a series of discrete regions bearing capture nucleotide probes which are able to bind by hybridisation to corresponding target nucleotide sequences. If the last ones are labelled, a signal can be detected and measured at the binding location and its intensity gives an estimation of the amount of target sequences present in the sample.

### State of the art

The Company Affymetrix has developed a method for direct synthesis of oligonucleotides on a support. The presence of a particular sequence at a specific location is obtained by using masks at each step comprising the addition of a new nucleotide on a growing oligonucleotide in order to obtain the desired sequence. This mask technique is derived from the photolithographic technology and is coupled with the use of photoprotective groups, which are released before a new nucleotide is added (EP-A1-0476014, US-A-5,445,934, US-A-5,143,854 and US-5,510,270) . The obtained array can contain several thousands of discrete domains, each one bearing a specific oligonucleotide as capture probe for the possible detection of thousands of target sequences. Furthermore, only small oligonucleotides are present on the surface, and find applications mainly for sequencing or identifying sequences in a pattern of positive spots corresponding to each specific oligonucleotide present on the array. The identification of the target sequence is thereafter made by comparison of said pattern with a reference. Said technique was applied to the identification of Mycobacterium tuberculosis rpoB gene (WO97/29212 and WO98/28444), wherein the capture probe comprises less than 30 nucleotides. Said method allows an analysis of two different sequences that may differ by a single nucleotide. In this last method, small capture probes (between 10 and 20 nucleotides) are preferred since the discrimination between two oligonucleotides differing in one base is higher when their length is smaller. Said last method is especially used for the identification of Single Nucleotide Polymorphism (SNP) or for genotyping.

However, for longer target fragments (like the cDNA copy of mRNA), several fragments can recognise the same probe and the rate of hybridisation is lower. Therefore, the fragments should be cut into smaller species and the method should require the use of several capture probes in order to obtain a pattern of signals which attest the presence of a given gene (WO97/10364 and WO97/27317). In this case, short capture sequences are used with few nucleotides, preferably about 45 base pairs, and in practice, between 10 and 25 nucleotides per probe.

The document US-5,605,662 describes the binding of full nucleotide sequences on plots of electronic based chips, each of the plots bearing a specific oligonucleotide sequence. In order to obtain an SNP determination, a target sequence is spotted on the chips and specific small labelled nucleotides are then incubated in order to determine their fixation on the target. A change in the plot charge allows a discrimination of the different nucleotide differing in one base, which allows a specific SNP analysis (Gilles et al. 1999, Nature Biotechnology 17, p. 365 (1999)).

The document WO94/22889 describes electronic chips made of preconstructed oligonucleotides bearing a pyrol group used in an electropolymerisation step and allows the formation of a conducting polymer. This method is particularly suitable for the preparation of electronic chips bearing small capture probes.

Long capture probes can be attached to several surfaces and be used in arrays in order to hybridise target sequences.

The approaches using either small or long capture probes fixed on array, are not optimal conditions when applied for diagnostic purpose, especially, when different sequences having a high homology between them should be detected and possibly quantified at the same time upon the same array.

### Aims of the invention

The present invention aims to provide a new method to improve the detection and possibly the quantification of nucleotide sequences, preferably multiple homologous sequences, coming from different or the same organism by hybridisation on single stranded capture probes, which does not present the drawbacks of the state of the art.

### Summary of the invention

The inventors have discovered that it is possible to provide a sensitive detection method (possibly combined with a quantification) of multiple nucleotide sequences upon an array even if said multiple sequences are homologous and are present simultaneously in the sample submitted to the analysis. Until now, it was impossible to obtain such a sensitive detection, when homologous sequences had to be analysed simultaneously. Either the sensitivity was low or null or there was cross-reactivity of the different (but homologous) target sequences on the same capture probes.

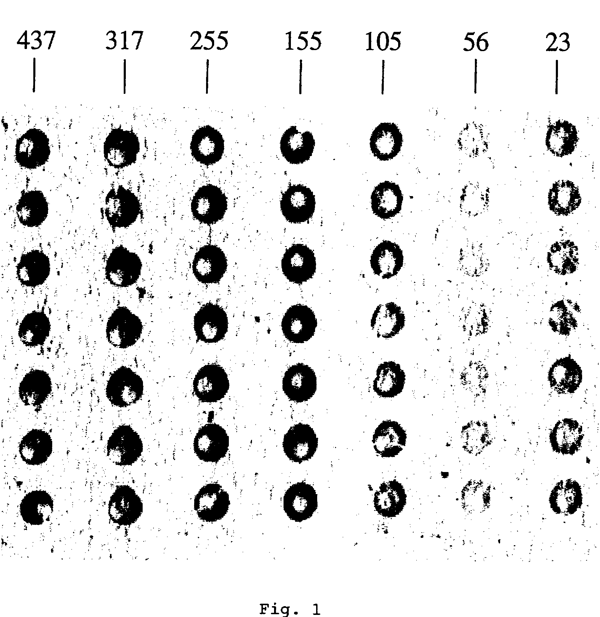

One unexpected observation was the result obtained when a given target sequence was incubated with an array bearing corresponding capture probes but of various length (example 2 and figure 1). With a target sequence made of a double stranded amplicon (just denatured before the experiment), either the use of too long or too small capture probes gave a lower signal and sometimes a much lower signal compared to the one obtained when capture probes have the same length as the target sequence. The highest sensitivity was obtained with capture probe with moderate length and a size similar to the target amplicon. For very long target amplicons, the situation is much more complicated (reassociation of the amplicons, secondary structures, ...) and depends on the nature of the sequence. In sandwich hybridisation of long target amplicons upon microplates, optimal capture probes have been found to be between 50 and 500 bases (WO98/11253).

The present invention is related to an (improved in sensitivity and specificity compared to known techniques) detection and/or quantification method of multiple target nucleotide sequences, possibly homologous to each other(s), said nucleotide sequences being present in a biological sample to be analysed and said method comprising the steps of:
- amplifying or copying at least a part (or portion) of the original nucleotide sequences (to be detected and/or quantified in the biological sample) into target nucleotide sequences,
- possibly denaturing the double stranded target nucleotide sequences into single target nucleotide sequences,
- possibly cleaving said sequences into smaller target nucleotide sequences having a length higher than about 40 and lower than about 400 base pairs (or at a preferred size described hereafter) by various methods (restriction enzymes, addition of NaOH, etc.),
- putting into contact the obtained target nucleotide sequences with corresponding capture nucleotide sequences bounded (fixed) to an insoluble solid support, said capture nucleotide sequences having a single strand length comprised between about 40 and about 400 base pairs, preferably between 50 and 350 base pairs, more preferably between 100 and 300 base pairs, even more preferably between 120 and 200 base pairs, said capture nucleotide sequences being disposed upon the insoluble solid support according to an array with a density of at least 5 different capture nucleotide sequences bounded to said solid support/cm² surface of solid support.

The method comprises also the step of detecting and/or possibly quantifying a signal resulting from the formation of double stranded nucleotide sequences resulting from their hybridisation by base pairing.

The method according to the invention may also further comprise the step of correlating the signal of detection to the presence of specific microorganisms, or genetic characteristics (polymorphism, genetic diseases, diagnostic and monitoring of cancer, etc.) for a patient (from which the biological sample has been obtained). The biological sample can be any culture medium wherein microorganisms or pollutants are present, or an extract obtained from a plant or an animal organ, tissue, cell or biological fluid obtained from a patient, including a human.

The various steps of the method according to the invention can be done by the person skilled in the art with various means and methods well known by the person skilled in the art and described in the literature.

The method according to the invention can be performed by using a specific diagnostic and/or quantification kit comprising at least an insoluble solid support upon which capture nucleotide sequences are disposed (preferably bounded to the solid support by a covalent link) according to an array with a density of at least 5 different capture nucleotide sequences / cm² surface of the insoluble solid support, said capture nucleotide sequences having a length comprised between about 40 and about 400 base pairs or preferably a length as above-described.

In the method and kit according to the invention, the density of the capture nucleotide sequences upon the array of the solid support can be increased, for instance by having more than 10, 20, 50, 100 or more than 1000 capture nucleotide sequences / cm² surface of solid support.

The kit according to the invention may also incorporate various media or devices for performing the method according to the invention. Said kit can also be included in an automatic device such as a high throughput screening apparatus for the detection and/or the quantification of multiple nucleotide sequences present in a biological sample to be analysed. Said kit or apparatus can be adapted for performing all the steps or only several specific steps of the method according to the invention.

In the method, kit or apparatus according to the invention, the length of the single stranded capture nucleotide sequences is preferably identical to the length of the target nucleotide sequences to be detected and/or quantified or may differ, preferably by less than 50% in total length, more preferably less than 30% in total length, even more preferably less than 10% in total length.

The method, kit or apparatus according to the invention are suitable for the detection and/or the quantification of target nucleotide sequences which are made of DNA or RNA, including sequences which are partially or totally homologous upon their total length.

The method according to the invention can be performed even when the different target nucleotide sequences present between them an homology greater than 30%, greater than 60% and even greater than 80%.

In the method, kit or apparatus according to the invention, the capture nucleotide sequences are advantageously covalently bounded (or fixed) upon the insoluble solid support, preferably by one of their extremities as described hereafter.

With the method according to the invention, the yield of hybridisation is advantageously greater than 10%, preferably greater than 50%, or can achieve almost 100%.

It is also clear for the person skilled in the art that the method, kit and apparatus according to the invention may comprise the use of other bounded capture nucleotide sequences (i.e. by allowing an hybridisation with a standard sequence used for the quantification, with a consensus sequence of different micro-organisms strains or with a sequence allowing a detection of a possible an antibiotic resistance by micro-organisms) or other non-homologous sequences, said other capture nucleotide sequences having possibly a length higher than 400 base pairs and being also bounded upon the insoluble solid support (biochip), preferably in the array made with the other bounded capture nucleotide sequences.

The solid support according to the invention can be or can be made with materials selected from the group consisting of glasses, electronic devices, silicium or plastic support, compact discs, filters, metallic supports or a mixture thereof. Advantageously, said solid support is a single glass plate which may comprise additional means (barcodes, markers, etc.) or media (counting, etc.) for improving the method according to the invention.

The amplification step(s) used in the method according to the invention is advantageously obtained by well known amplification protocols, preferably selected from the group consisting of PCR, LCR, CPR, NASBA, ICR or Avalanche DNA techniques.

Advantageously, the target nucleotide sequences to be detected and/or quantified are labelled previously to their hybridisation with the capture nucleotide sequences. Said labelling (with known techniques from the person skilled in the art) is preferably also obtained upon the amplified sequence previously to the denaturation (if the method comprises an amplification step).

Advantageously, the length of the target nucleotide sequences to be detected is determined by the conditions of the above-identified amplification protocols or determined by the use of specific primers for a retro-transcription of the 3' or 5' end of the original biological nucleotide sequences to be detected and/or quantified, especially if it is a RNA sequence. The length of said target nucleotide sequences to be detected and/or quantified can be also determined by the use of specific primer and blocking probes for the retro-transcription of the original biological nucleotide sequences (especially for RNA sequences). Advantageously, said RNA sequences are 16S and 23S rRNA sequences or 18S and 28S rRNA sequences.

Given the possibility to break DNA strands, the amplified or copied sequences can be broken into fragments which fulfil the requirement here mentioned.

In a preferred embodiment of the present invention, the target nucleotide sequences to be copied or amplified are obtained from different parts or portions of the corresponding (homologous) DNA or RNA original biological nucleotide sequence.

In a preferred embodiment of the invention, the primers used for the amplification preferably bear at their 3' end a base specific of one of the homologous sequences.

According to a further aspect of the present invention, the method, kit or apparatus according to the invention is advantageously used for the detection and/or the quantification of different Staphylococci species or variants, preferably the Staphylococcus aureus, the Staphylococcus epidermidis, the Staphylococcus saprophyticus, the Staphylococcus hominis or the Staphylococcus haemolyticus present together or separately in a biological sample, said detection being obtained by detecting the genetic variants of the FemA gene in said different species, preferably by using specific locations in the FemA genetic sequence, as described in the document WO99/16780 incorporated herein by reference.

Preferably, the primers and the specific portions of said FemA sequence used for obtaining amplified products are the ones described hereafter in the examples.

The method according to the invention may also comprise means for obtaining a quantification of target nucleotide sequences by using a standard nucleotide sequence (external or internal standard) which can be brought into contact with the capture probes bounded upon the array of the solid support in the same conditions as said target nucleotide sequences (possibly after amplification or copying); the method comprising a step of quantification of a signal resulting from the formation of a double stranded nucleotide sequence formed by complementary base pairing between the capture nucleotide sequences and the standard nucleotide sequences and a step of a correlation analysis between the signal resulting from the formation of said double stranded nucleotide sequence and the signal resulting from the double stranded nucleotide sequence formed by complementary base pairing between capture nucleotide sequences and target nucleotide sequences in order to quantify the presence of the original nucleotide sequence to be detected and/or quantified in the biological sample. The characteristic of standard nucleotide sequences used according to the invention can be found in the document WO98/11253 incorporated herein by reference. Said standard nucleotide sequence (external and/or internal standard) is advantageously included in the kit or apparatus according to the invention, possibly with the media and device(s) necessary for performing the different steps according to the invention, such as the hybridisation and culture media, polymerases, enzymes, standard sequences and labelling molecules.

The present invention will be described in details in the following non-limiting examples in reference to the enclosed figures.

### Brief description of the drawings

Fig. 1 shows the influence of the capture probe length on the yield of hybridisation of target amplicons. The target sequences were 155 bases long and 100 fmoles were incubated on a chips containing single stranded capture probes going from 23 to 437 bases long. The length of the capture probe is shown on the figure.

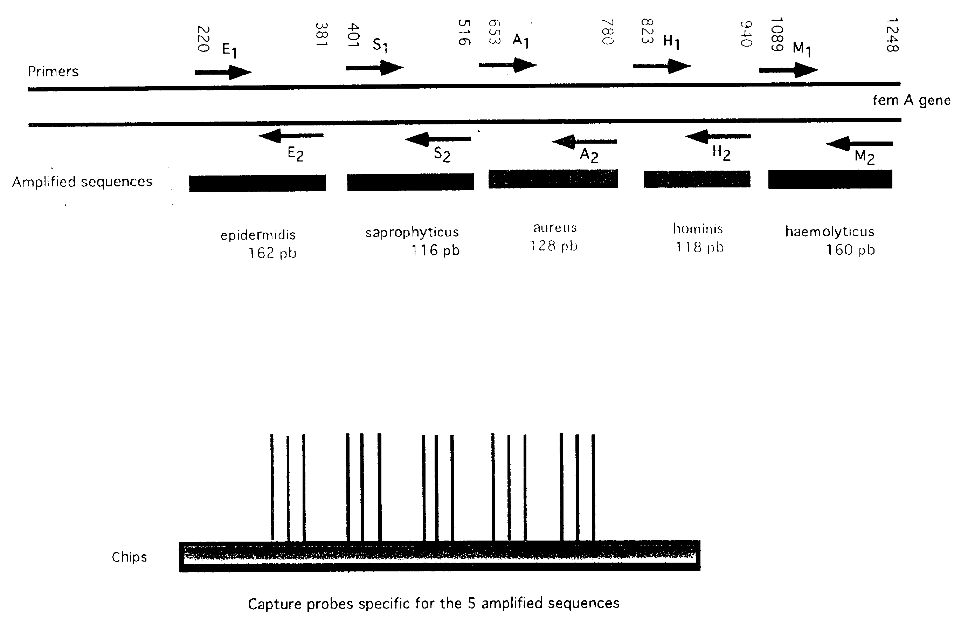

Fig. 2 schematically represents the FemA detection of 5 different species of Staphylococci. The locations of the 5 pairs of primers used for specific amplification of one of the 5 different sequences belonging to the 5 Staphylococci species are shown on the FemA sequence. A consensus sequence is also amplified by using 2 primers common to all Staphylococci species. The labelled amplified sequences are then hybridised on the chips.

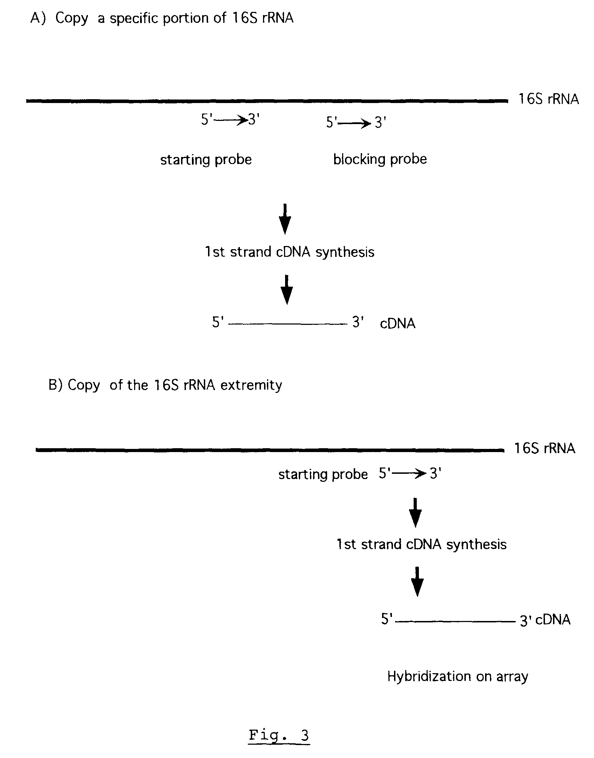

Fig. 3 schematically represents the detection of rRNA by the copy of small portions of the sequence either at its 5' end using one starting probe or along its sequence using both a starting probe and a blocking probe. The small copied sequences are then hybridised on the array of the chips.

### Definitions

The term "nucleoside triphosphate" refers to nucleosides present in either DNA or RNA and thus includes nucleosides which incorporate adenine, cytosine, guanine, thymine and uracil as bases, the sugar moieties being deoxyribose or ribose. Other modified bases capable of base pairing with one of the conventional bases adenine, cytosine, guanine, thymine and uracil may be employed. Such modified bases include for example 8-azaguanine and hypoxanthine.

The term "nucleotide" as used herein refers to nucleosides present in nucleic acids (either DNA or RNA) compared with the bases of said nucleic acid, and includes nucleotides comprising usual or modified bases as above described.

References to nucleotide(s), oligonucleotide(s) and the like include analogous species wherein the sugar-phosphate backbone is modified and/or replaced, provided that its hybridisation properties are not destroyed. By way of example the backbone may be replaced by an equivalent synthetic peptide, called Peptide Nucleic Acid (PNA).

The primer sequence need not reflect the exact sequence of the template to be amplified or copied provided that under hybridising conditions the primers are capable of fulfilling their stated purpose in a genetic amplification. Mismatched bases can be introduced into the primer sequence to provide altered hybridisation introduced into the primer sequence to provide altered hybridisation stringency. This is especially important for amplification or copying of the same regions of homologous sequences by the same primers as mentioned in this invention. Commonly, however, the primers have exact complementarity except in so far as non-complementary nucleotides may be present at a predetermined primer terminus.

The terms "hybridising specifically to", refer to the binding, duplexing, or hybridising of a molecule only to a particular nucleotide sequence under stringent conditions when that sequence is present in a complex mixture (e.g., total cellular DNA or RNA). The term "stringent conditions" refers to conditions under which a probe will hybridise to its target subsequence, but not to other sequences. Stringent conditions are sequence-dependent and differ according to circumstances. An example of stringent conditions is described by Sambrook et al., §§ 9.47-9.51 in *Molecular Cloning : A Laboratory Manual,* Cold Spring Harbor, Laboratory Press, Cold Spring Harbor, New York (1989).

The term "background" refers to hybridisation signal resulting from non-specific binding, or other interactions, between the labelled target nucleic acids and components of the oligonucleotide array (e.g., the oligonucleotide probes, control probes, the array substrate, etc.). A single background signal can be calculated for the entire array, or a different background signal may be calculated for each spot. In a preferred embodiment, background is calculated as the average hybridisation signal intensity produced by hybridisation to probes that are not complementary to any sequence found in the sample. Background can also be calculated as the average signal intensity produced by uncoloured regions of the array.

The term "quantifying" can refer to absolute or to relative quantification. Absolute quantification is obtained by inclusion of known concentration(s) of one or more target nucleic acids (e.g. control nucleic acids or with known amounts of target nucleic acids themselves) and referencing the hybridisation intensity of unknowns with the known target nucleic acids (e.g. through generation of a standard curve). Alternatively, relative quantification is obtained by comparison of hybridisation signals between two or more target sequences or as a relative intensity of one spot compared to the overall spots, e.g. comparison to signals generated by the hybridisation of target issued from housekeeping genes such as GAPDH. The quantification is preferably applied when comparing changes in samples or tissues in various experimental conditions.

"Homologous sequences" mean nucleotide sequences having the same nucleotide at corresponding positions. They are generally defined as a minimum of homology (or sequence identity) between sequences wherein the percentage of identical nucleotides after the sequences has been optimally aligned taking into account additions or deletions like gaps in one of the two sequences to be compared. This is the case for sequences of a given gene but present in genetically different sources like different organisms or for proteins or enzymes of the same family. The degree of homology (or sequence identity) can vary a lot as homologous sequences may be only in one part, a few parts or portions or all along their sequences. The parts or portions of the sequences that are identical in both sequences are said conserved. The sequences showing a high degree of invariance in their sequences are said to be highly conserved and they present a high degree of homology.

Methods of alignment of sequences are based on local homology algorithms which have been computerised and are available as for example (but not limited to) Clustal^{®}, (Intelligenetics, Mountain Views, California), or GAP^{®}, BESTFIT^{®}, FASTA^{®} and TFASTA^{®} (Wisconsin Genetics Software Package, Genetics Computer Group Madison, Wisconsin, USA) or Boxshade^{®}.

### Detailed description of the invention

The amplification of target sequences of 100 to 200 bases and hybridisation on capture probes comprised between 100 and 200 bases allows to solve many problems posed by the differential diagnostic of homologous sequences. However smaller lengths (especially for capture probes) going down to 40 bases and longer length going up to 400 bases can also be used (mostly limited by the amplification step which needs the use of two primers and some sequences to be copied). The reduction of the capture probe for a given target sequence leads to a decrease in sensitivity, but an increase in specificity. Longer target sequences limit the possibility to design the various targets amplicons on the same sequence as shown in Fig. 2. It also reduces specificity and can lead to secondary structures decreasing the yield of hybridisation.

Target DNA sequences are amplified by classical methods like PCR using primers so that small fragments are produced which are then used for hybridisation on an array bearing the corresponding capture probes. RNA can be copied and retro-transcripted in cDNA and amplified the same way if necessary. Short sequences of DNA can also be obtained by specific cleavage with restriction enzymes and small sequences of RNA by non specific cleavage by heating or in the presence of a base (WO97/10365).

When homology between the sequences to be detected in the sample is not too high (typically between 10 and 60%), the homologous sequences are amplified or copied (for instance by using the same primers) and the discrimination is obtained on the array using moderate length capture probes. The use of the same primers is possible given that some parts of the sequences are similar or even identical on a certain distance and it facilitates the amplification step. However since the sequences are also rather different makes possible to capture them specifically on moderate length capture probe. The fact that both target and capture probes are of similar length makes the detection sensitive.

However when several sequences with high homology (for instance higher than 60%) have to be analysed the discrimination by hybridisation on capture probes only is not always sufficient (cross-reactions between two homologous sequences on the same capture probe can be observed). Therefore, different small fragments of the target sequences are either amplified or copied. These fragments are located in different part of the sequence for the different targets. These small fragments are then detected on their specific capture probes of moderate length. High sensitivity is obtained throughout the use of capture probes of moderate length similar to the sequences to detect. The specificity is obtained first during the amplification using specific primers for each sequence to be amplified or copied and secondly on the array (since each capture probe correspond to a specific sequence) located at specific places along the target sequences. An illustration of the detection of FemA genes in 5 different Staphylococcus is presented in Fig. 2 and in example 4. For a sample analysis, the different primers are used together in a multiplex PCR amplification before analysis on the biochips. Preferably, one may use primers specific for each target having also at their 3' end a base specific of the sequence to detect so that (in appropriate conditions) only the target sequence bearing the corresponding base will be copied and amplified by the polymerase.

In an alternative, a semi-consensus multiplex PCR was tested by using a primer common for all sequences and a second primer specific of each homologous sequence. Specific capture probes of moderated length were selected for each sequence in a different part of the overall target sequence.

In another alternative, which can be combined with the previous mentioned ones, multiple capture probes which recognise different parts of a same target are fixed to the array, which contain two or three capture probes binding to different parts of a given amplified sequence. The resulting signals for each of these spots are in a given ratio for a given target sequence, while it is different for another sequence which partly cross-reacts as the homology between two sequences is not homogeneous (with some parts being very different while others are more conserved). This redundancy of the signal can be useful when qualitative data are requested. However, it lowers the signal intensity since the lengths of the capture probes are smaller than the target to be bound.

The assay for RNA present in large amount like the 16s or 23s rRNA in procaryote cells or the 18s or 28s rRNA in eucaryote cells does not require necessarily amplification (RNA sequences are present in multiple copies in cells). The detection based on their hybridisation on array is particularly well suited since they show a high degree of homology between different species. The extremity of the RNA is copied by retro-transcription (by using one primer specific of each sequence or common to some or all of them), that will hybridise at a short distance from the extremity (example 5). However it is also possible to copy one specific part of the RNA by using a primer to start the copy and a blocking probe which binds to the RNA and stops the copying by the reverse transcriptase (example 6). This blocking oligonucleotide has its 3' end blocked so that it can not be used by the transcriptase to start a copy. The most simple 3' block being a deoxy 3' carbon but others like the presence of a pyrophosphate at the 3' carbon or a 2',3'-dideoxycarbon are also working. The 5' end of this blocking oligonucleotides is also modified like for example by a NH₂ group in order to block transcriptase. The primer can also bear at its 3' end a base specific of a given sequence, in order to make a more specific copy. Therefore, different specific parts of the rRNA of different organisms can be copied making their detection on the array unequivocal since not only their sequence, but also their location will be specific.

The sme copy of the RNA can be obtained with messenger RNA (mRNA) which can be transcripted in cDNA and amplified by PCR if necessary.

### Arrays

The development of chemistry allows the covalent fixation to glass of nucleotides bearing a specific functional group (Lamture et al., Nucleic Acid Res. 22, pp. 2121-2125 (1994)). For instance, by using aminoterminal groups present on the oligonucleotides, it is possible to obtain a covalent binding of said nucleotides upon aldehyde groups present on the array. All the steps of the process can be very well controlled and easily adapted for a scaling up production and the net result is the obtention of attached single stranded capture probes at a given density with well defined length available for the hybridisation requirement. The capture probes are either chemically synthesised or produced by PCR (amplicons) and bounded to a functionalised glass by a robot and thereafter rendered single stranded. Capture probes of any sequence and of any length going from the smallest to the largest sequences (i.e. from 10 to 1,000 nucleotides), can be attached to the glass. The capture probes have complementary sequences related to the target DNA to detect and have a similar if not identical length. Difference of 50 % in the length still gives a high sensitivity binding. The array is constructed with an appropriated automate which deposits the capture probes at a given location which delimitates a spot. The automatisation allows a high precision intended for industrial production of reproducible array. The number of spots depends of the number of target sequences to be detected. Triplicate of each capture probe plus the negative and positive controls (and when possible standard sequences) are used. The array contains typically between 20 and 100 spots, but arrays going to 400 spots and even higher than 1000 spots or more are possible. An array with 400 spots per cm² is obtained with pins of 0.2 mm at low cost. As the capture probes are present in a sufficient number, it allows the lecture of these spots with a great resolution with known detection apparatus.

Other supports and methods for binding of capture probes on the surface of an array can also be used or adapted from known techniques. Filters either of nitrocellulose or nylon are also proposed for arrays. The binding sites of the DNA on these filters is however at random along the sequence and it is impossible to predict the size of the available single stranded sequence which is then available for hybridisation.

Fixation of oligonucleotides sequences can be obtained through covalent or non-covalent binding on these supports in a direct or indirect reaction. One common method is to spot the capture probes on a surface where polylysine has been attached on glass or plastic. Fixation can also be obtained through the binding of biotinylated oligonucleotides on streptavidin coated surfaces or through the use of proteins with binding affinity for oligonucleotides (EP-A-0491059).

Gel layers containing the capture probes have been proposed by Mirzabekov (US-A-5,552,270 and EP-0535242). Copolymerisation of acrylamide with vinyl bearing oligonucleotides has also been proposed (US-A-5,736,257). Other supports, which can be activated for covalent fixation of oligonucleotides, can be used for arrays. Plastic like polycarbonate as present on CD was activated in order to fix capture probes and be used as a bio-CD array (W099/35499).

### Hybridisation conditions

Hybridisation conditions have to be optimised according to the sequences to be detected. The hybridisation between two DNA or RNA chains is a complex process which is initiated by the binding of a few (4-5) nucleotides which recognised themselves in a specific way and once they are bound a very fast process thermodynamically favourable elongation of the binding occurs along the sequence. The binding is thus dependent both of kinetic and thermodynamic parameters and experimental conditions can be adapted in order to modify both of them. Temperature accelerates the kinetic process but there is an optimum for the temperature used to obtain a maximum binding of the target. The salt concentration modifies the stringency conditions: more salts present in the solution, more easy will be the binding of the two chains.

Preferred conditions are obtained with targets and capture probes of the same size and of moderate length. These conditions make the search for identical optimised hybridisation conditions rather easy since from a kinetic point of view, these targets sequences will behave in a similar way given there similar length. For binding affinity, sequences which are similar in their proportion of A/T compared to the G/C content affect the stability of the DNA duplex.

According to the invention, a very good yield of capture of the target on the capture probes can be obtained even if the targets are double stranded like after PCR amplification. The hybridisation of the target DNA is a competitive reaction: the target strand can hybridise on the fixed capture probe but can reassociate in solution with its complementary strand. The reaction in solution is always kinetically favourable due to the free movement of molecules in solution. The rate of reaction is proportional to the square root of the length of the shorter strand. Capture probes that have almost similar length as the target are used in this invention. The two reactions are independent of the length, since they are the same or similar. An optimum number of the same capture probes has been fixed per spot so that the high number of capture probes compensates for the loss obtained by the diffusion constrains. Moderated length capture probes disposed at a certain distance from the surface lower the diffusion effect.

Other components of reactive solutions have also to be incorporated like buffer(s), detergent(s), DMSO or the addition of non specific DNA like salmon DNA.

### Detection

After hybridisation on the array, the target sequences can be detected by current techniques. Without labelling, preferred methods are the identification of the target by mass spectrometry now adapted to the arrays (US-A-5,821,060) or by intercalating agents followed by fluorescent detection(W097/27329 or Fodor et al., Nature 364, p. 555 (1993)).

The labelled associated detections are numerous. A review of the different labelling molecules is given in W0 97/27317. They are obtained using either already labelled primer or by incorporation of labelled nucleotides during the copying or amplification step. A labelling can also be obtained by ligating a detectable moiety onto the RNA or DNA to be tested (a labelled oligonucleotide, which is ligated, at the end of the sequence by a ligase) . Fragment of RNA or DNA can also be incorporate labelled nucleotides at their 5'OH or 3'OH ends using a kinase, a transferase or a similar enzyme.

Labels like fluorescent probes like Cy3, Cy5 and Cy7 are suitable for analysing an array by using commercially available array scanners (General Scanning, Genetic Microsystem,...) . Radioactive labelling, cold labelling or labelling with small molecules recognised thereafter by specific ligands (streptavidin or antibodies) are common methods. The resulting signal of target fixation on the array is either fluorescent, colorimetric, diffusive, electroluminescent, bio- or chemiluminescent, magnetic, electric like impedometric or voltametric (US-A-5,312,527). The two preferred embodiments of the invention are the fluorescent detection or the gold labelling of the bound target in order to obtain a precipitate or silver staining which is then easily detected and quantified by a scanner (EP-99870106.4).

### Quantification

The signal obtained for each spot is recorded and the mean of the signal is calculated for identical capture probes. In practice at least two and preferably three to five identical spots are present on each array in order to correct for variation which can occur at any step of the process. The background value is identified either in the part of the array which has no capture probe or on spots bearing non specific capture probe (negative control). A positive control is preferably added (a DNA sequence which is added to the hybridisation solution and in which capture probe is present at least on one spot of the array). The positive control allows to test for the hybridisation step, the solutions and conditions used and the detection. Different positive probes present at various concentrations can also be added to the sample in order to obtain a reference curve for the signal. The various signals of the spots can then be compared to this reference curve.

Quantification has to take into account not only the hybridisation yield and detection scale on the array (which is identical for target and reference sequences) but also the extraction, the amplification (or copying) and the labelling steps. Internal standard are used in quantification by the measurement of the target sequence compared to a given sequence (reference) and to which the other values will be compared.

External standard can also be added to the sample for the quantification. If PCR is used, an internal standard contains at its extremities the same sequences as the target in order to be amplified by the same primers. It can also be of the same length, has the same GC content or even have a large part of its sequence identical to the target in order to be really competitive during the amplification step.

### Examples

### Example 1 : Detection of target nucleotides on an array

### Capture probe immobilisation

The protocol described by Schena et al (Proc. Natl Acad. Sci. USA 93, 10614 (1996)) was followed for the grafting of aminated DNA to aldehyde derivatised. The long aminated capture probes (100-400 bases) were spotted at a concentration of 150 nM while the small oligonucleotides were at 450 nM. The capture probes were printed onto the silylated microscopic slides with a home made robotic device. We used 250 µm pins from Genetix (UK) and silylated (aldehyde) microscope slides from Cell associates (Houston, USA). The spots have 400 µm in diameter and the volume dispensed is about 1 nl. Slides were dried at room temperature and stored at 4 °C until used.

### Hybridisation

5 µl of hybridisation solution were loaded on glass slides bearing the capture probes. This mixture contained : SSC2X, SDS 4%, salmon sperm DNA 100 µg/ml, 2 nM biotinylated CMV amplicons of 437 bp and 10 nM of biotinylated target amplicons. Microarrays were covered with coverslips prewashed with ethanol 100%. Slides were denatured at 95 °C for 5 min. The hybridisation was carried out at 65° for 2 h. Samples were washed 4 times with Maleic buffer 10 mM pH 7.5, NaCl 15 mM, Tween 0.1%.

### Silver staining detection

The glass samples were incubated 45 min at room temperature with 800 µl of streptavidin labeled with colloidal gold. After washing the presence of gold served for catalysis of silver reduction using the staining revelation solution (Sigma St Louis, Mi) The slides were dried before being store at room temperature and analysed using a micro array reader.

### Example 2: Comparison of the sensitivity obtained for hybridisation of a target sequence of medium size (155 bp) on capture probes of various length

The protocols for capture probes immobilisation and silver staining detection are described in example 1. The capture probes and target DNA were obtained by amplification of CMV sequence by PCR using the following primers:

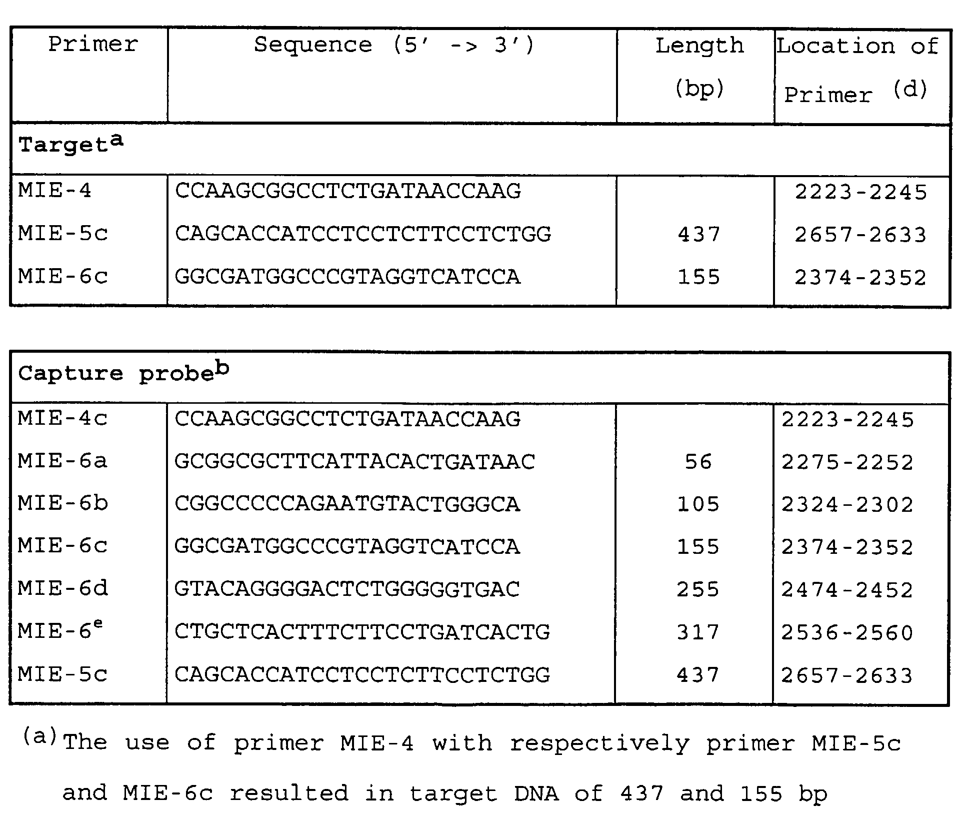

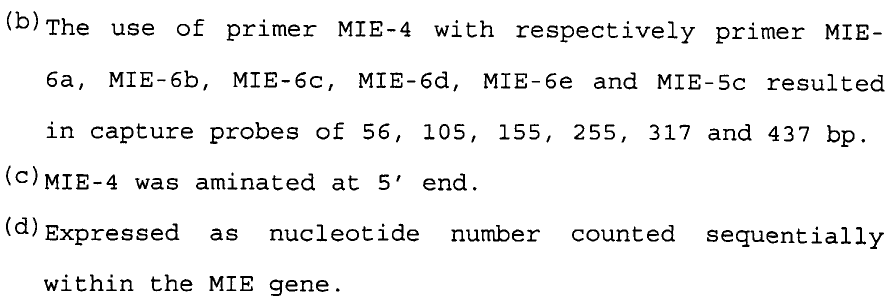

### Amplification

Plasmid pAT153-E (containing the exon 4 of the MIE gene of HCMV DNA AD169 strain) was amplified by PCR. The amplification was performed in a 100 µl volume containing 1.5 mM MgCl₂, 10 mM Tris pH 8.4, 50 mM KCl, 1 µM of each primer, 100 µM of each dNTP, 2.5 U of Taq DNA polymerase Gold and 10 ng of plasmid pAT153-E. Samples were first denatured at 94 °C for 10 min to activate the polymerase. Then 40 cycles of amplification were performed consisting of 30 sec at 94 °C, 30 sec at 65 °C and 30 sec at 72 °C and a final extension step of 10 min at 72 °C. Water controls or 100 copies of plasmid DNA were used respectively as negative or positive controls of the amplification.

PCR of target DNA also includes 100 µM of biotin-16-dUTP.

The hybridisation step was performed as described in example#1. The hybridisation was carried out at 65 °C for 2 h in the presence of 100 fmoles of biotinylated target DNA.

### Example 3: Influence of the capture probe length on the yield of fixationof long target amplicons (437 bp)

The experiment was conducted as described in example 3 but with a target of 437 bp. This target sequence was obtained by amplification of the Plasmid pAT153-E using the primer MIE-4 and the primer MIE-5c. Hybridisation was conducted on the biochops as in example 3.

### Example 4: Detection of FemA sequences from different bacterial species on the same array

The FemA genes corresponding to the different Staphylococcus species were amplified separately by multiplex PCR using the following primers:

S. aureus 1 : 5′ CTTTTGCTGATCGTGATGACAAA 3′
S aureus 2 : 5′ TTTATTTAAAATATCACGCTCTTCG 3′
S. epidermidis 1 : 5′ TCGCGGTCCAGTAATAGATTATA 3′
S. epidermidis 2 : 5′ TGCATTTCCAGTTATTTCTCCC 3′
S. haemolyticus 1 : 5′ CTATGGTATTAGCGGTAATTTTAG 3′
S. haemolyticus 2 : 5′ TTTAATCTTTTTGAGTGTCTTATAC 3′
S. saprophiticus 1 : 5′ TAAAATGAAACAACTCGGTTATAAG 3′
S. saprophiticus 2 : 5′ AAACTATCCATACCATTAAGTACG 3′
S. hominis 1 : 5′ CGACCAGATAACAAAAAGCACAA 3′
S. hominis 2 : 5′ GTAATTCGTTACCATGTTCTAA 3′

The location of these primers and their specificity for the different Staphylococcus are presented in figure 2.

The multiplex PCR was performed in a final volume of 50 µl containing: 1.5 mM MgCl₂, 10 mM Tris pH 8.4, 50 mM KC1, 0.8 µM of each primer, 50 µM of each dNTP, 50 µM of biotin-16-dUTP), 1.5 U of Taq DNA polymerase Biotools, 7.5% DMSO, 5ng of plasmid containing FemA gene. Samples were first denatured at 94 °C for 3 min. Then 40 cycles of amplification were performed consisting of 30 sec at 94 °C, 30 sec at 60 °C and 30 sec at 72 °C and a final extension step of 10 min at 72 °C. Water controls were used as negative controls of the amplification. The sizes of the amplicons obtained using these primers were 116 bp for *S. saprophiticus,* 128 bp for *S*. *aureus,* 118 bp for *S. hominis,* 162 pb for *S*. *epidermidis* and 160 bp for *S. haemolyticus.*

The sequences of the capture probes were the same as the corresponding amplicons but they were single strands.

Protocols for capture probes immobilisation, hybridisation and silver staining detection are described in example 1.

### Example 5: Detection of 16S rRNA from different bacteria bycopy of its extremity

The copy of the 16S rRNA extremity was done on 2 µg of total RNA extracted from bacteria using the following procedure.

In a sterile, nuclease free microtube, 1 ug of the probe was added to the RNA. Nuclease free water was used to achieve a final volume of 15µl.

The probes used were either species-specific or universal. The following sequences were used for the three bacteria and as universal probe:

Starting probe:
Escherichia coli 5' ATTAACTTTACTCCCTTCC 3'
Bacteroides distasonis 5' GACACGTCCCGCACTTTZ 3'
Bifidobacterium longum 5' CGCTTGCTCCCCGAZTAAA 3'
Universal probe 5' GTATTACCGCGGCTGCTG 3'

The mixture was denatured for 5 min at 70 °C and then chilled on ice for 5 min. The reverse transcription was performed by adding the following components to the annealed probe /template : 5 µl of 5X AMV RT Buffer (250 mM Tris-HCl pH 8.3, 250 mM KCl, 50 mM MgCl₂ 50 mM DTT and 2.5 mMSpermidine), 40 units of Rnasin ribonuclease inhibitor( Promega, Madison, US), 1 mM dATP, 1 mM dCTP, 1 mM dGTP, 0.65 mM dTTP, 0.35 mM biotin dUTP and 30 units of AMV RT( Promega, Madison, US). The final volume was adjusted to 25 µl with nuclease free water. The reaction mixture was mixed gently and incubated for 60 min at 60 °C.

The single strand DNA obtained from different bacteria was hybridised and detected on an array. The protocol used is described in the first example.

### Example 6: Detection of 16S rRNA from different bacteria by copying of a small portion of the sequence

A specific sequence of the 16S rRNA sequence of E. *coli* was copied using both a starting probe and a blocking probe which hybridises on the 16S rRNA and stops the reverse transcription. The following sequences were used as starting and blocking probes:

Starting probe 5' CTCTGAAAACTTCCGTGGATG 3'
Blocking probe 5' GTATCTAATCCTGTTTGCTCCCCACGCT 3'

In a nuclease free microtube, 0.5 µg of the starting probe and 2 µg of the blocking probe were added to 2 µg total RNA extracted from bacteria. Nuclease free water was added to a final volume of 15 µl. The reverse transcription was conducted as in example 5.

The single strands cDNA obtained from the different bacteria were hybridised and detected on the array following the protocol described in example 1.

## Claims

1. Detection and/or quantification method of multiple target nucleotide sequences, homologous to each other(s), and being possibly present in a biological sample, comprising the steps of:
- amplifying or copying at least a part of original nucleotide sequences present in the biological sample into target nucleotide sequences;
- putting into contact the obtained target nucleotide sequences with corresponding capture nucleotide sequences bounded to an insoluble solid support, characterised in that said capture nucleotide sequences have a (single stranded) length comprised between about 40 and about 400 base pairs, and in that said capture nucleotide sequences are bound to the insoluble support according to an array with a density of at least 5 different capture nucleotide sequences / cm² surface solid support, and
- detecting and/or quantifying a signal resulting from the formation of double stranded nucleotide sequences resulting from their hybridisation by complementary base pairing.

2. Method according to claim 1, characterised in that the length of the capture and target nucleotide sequences is comprised between 50 and 300 base pairs, preferably between 100 and 200 base pairs.

3. Method according to any of the preceding claims, characterised in that the length of the capture nucleotide sequences differs less than 50% to the length of the target nucleotide sequences.

4. Method according to any of the preceding claims, characterised in that the different target nucleotide sequences to be detected present an homology between each other higher than 30%, preferably higher than 60%, more preferably higher than 80%.

5. Method according to any of the preceding claims, characterised in that the insoluble solid support is selected from the group consisting of glasses, electronic devices, silicium supports, plastic supports, compact discs, filters, metallic supports or a mixture thereof.

6. Method according to any of the preceding claims, characterised in that the length of the target nucleotide sequences to be detected and/or be quantified is determined by the use of specific primers for the retro-transcription or amplification of the 3' end of the original RNA or DNA nucleotide sequences to be detected and/or quantified in the biological sample.

7. Method according to any of the preceding claims 1 to 5, characterised in that the length of the target nucleotide sequences to be detected and/or quantified is determined by the use of specific primer and blocking probe for the retro-transcription of the original RNA nucleotide sequences to be detected and/or quantified in the biological sample.

8. Method according to any of the preceding claims, characterised in that the original nucleotide sequences to be detected and/or quantified in the biological sample are rRNAs, preferably selected from the group consisting of 16S, 23S, 18S and 28S rRNAs.

9. Method according to any of the preceding claims, characterised in that the original nucleotide sequences to be detected and/or quantified in the biological sample are FemA specific genetic sequences of Staphylococci species, preferably the FemA genes of the strain Staphylococcus aureus, epidermidis, saprophyticus, hominis and/or haemolyticus.

10. Diagnostic and/or quantification kit or apparatus, characterised in that it comprises an insoluble solid support upon which single stranded capture nucleotide sequences (allowing a specific hybridisation with target nucleotide sequences to be detected and/or quantified), are bounded, preferably by a covalent link, said single stranded capture nucleotide sequences being disposed upon the surface of the solid support according to an array with a density of at least 5 different single stranded capture nucleotide sequences / cm² surface of the solid support and in that said single stranded capture nucleotide sequences have a length comprised between about 40 and about 400 base pairs.


### Annex to the application documents subsequently filed sequences listing

SEQUENCE LISTING

<110> REMACLE, José

<120> METHOD AND KIT FOR THE SCREENING AND/OR THE
      QUANTIFICATION OF MULTIPLE HOMOLOGOUS NUCLEIC ACID
      SEQUENCES ON ARRAYS

<130> P.FNDP.05/EP

<140> 99870226.0
<141> 1999-10-28

<160> 26

<170> PatentIn Ver. 2.1

<210> 1
<211> 23
<212> DNA
<213> Artificial Sequence

<220>
<223> Description of Artificial Sequence: Target Primer

<400> 1
ccaagcggcc tctgataacc aag                                         23

<210> 2
<211> 25
<212> DNA
<213> Artificial Sequence

<220>
<223> Description of Artificial Sequence: Target Primer

<400> 2
cagcaccatc ctcctcttcc tctgg                                       25

<210> 3
<211> 23
<212> DNA
<213> Artificial Sequence

<220>
<223> Description of Artificial Sequence: Target Primer

<400> 3
ggcgatggcc cgtaggtcat cca                                              23

<210> 4
<211> 23
<212> DNA
<213> Artificial Sequence

<220>
<223> Description of Artificial Sequence: Capture Probe
      Primer

<400> 4
ccaagcggcc tctgataacc aag                                              23

<210> 5
<211> 24
<212> DNA
<213> Artificial Sequence

<220>
<223> Description of Artificial Sequence: Capture Probe
      Primer

<400> 5
gcggcgcttc attacactga taac                                             24

<210> 6
<211> 23
<212> DNA
<213> Artificial Sequence

<220>
<223> Description of Artificial Sequence: Capture Probe
      Primer

<400> 6
cggcccccag aatgtactgg gca                                              23

<210> 7
<211> 23
<212> DNA
<213> Artificial Sequence

<220>
<223> Description of Artificial Sequence: Capture Probe
      Primer

<400> 7
ggcgatggcc cgtaggtcat cca                                              23


<210> 8
<211> 23
<212> DNA
<213> Artificial Sequence

<220>
<223> Description of Artificial Sequence: Capture Probe
      Primer

<400> 8
   gtacagggga ctctgggggt gac                                               23


<210> 9
<211> 25
<212> DNA
<213> Artificial Sequence

<220>
<223> Description of Artificial Sequence: Capture Probe
      Primer

<400> 9
ctgctcactt tcttcctgat cactg                                            25


<210> 10
<211> 25
<212> DNA
<213> Artificial Sequence

<220>
<223> Description of Artificial Sequence: Capture Probe
      Primer

<400> 10
cagcaccatc ctcctcttcc tctgg                                            25

<210> 11
<211> 23
<212> DNA
<213> Artificial Sequence

<220>
<223> Description of Artificial Sequence: Staphylococcus
      aureus Primer

<400> 11
cttttgctga tcgtgatgac aaa                                              23


<210> 12
<211> 25
<212> DNA
<213> Artificial Sequence

<220>
<223> Description of Artificial Sequence: Staphylococcus
      aureus Primer

<400> 12
tttatttaaa atatcacgct cttcg                                            25


<210> 13
<211> 23
<212> DNA
<213> Artificial Sequence

<220>
<223> Description of Artificial Sequence: Staphylococcus
      epidermidis primer

<400> 13
tcgcggtcca gtaatagatt ata                                              23


<210> 14
<211> 22
<212> DNA
<213> Artificial Sequence

<220>
<223> Description of Artificial Sequence: Staphylococcus
      epidermidis primer

<400> 14
tgcatttcca gttatttctc cc                                                22

<210> 15
<211> 24
<212> DNA
<213> Artificial Sequence

<220>
<223> Description of Artificial Sequence: Staphylococcus
      haemolyticus Primer

<400> 15
ctatggtatt agcggtaatt ttag                                              24

<210> 16
<211> 25
<212> DNA
<213> Artificial Sequence

<220>
<223> Description of Artificial Sequence: Staphylococcus
      haemolyticus Primer

<400> 16
tttaatcttt ttgagtgtct tatac                                             25

<210> 17
<211> 25
<212> DNA
<213> Artificial Sequence

<220>
<223> Description of Artificial Sequence: Staphylococcus
      saprophyticus Primer

<400> 17
taaaatgaaa caactcggtt ataag                                             25

<210> 18
<211> 24
<212> DNA
<213> Artificial Sequence

<220>
<223> Description of Artificial Sequence: Staphylococcus
      saprophyticus Primer

<400> 18
aaactatcca taccattaag tacg                                              24


<210> 19
<211> 24
<212> DNA
<213> Artificial Sequence

<220>
<223> Description of Artificial Sequence: Staphylococcus
      hominis Primer

<400> 19
cgaccagata acaaaaaagc acaa                                              24


<210> 20
<211> 22
<212> DNA
<213> Artificial Sequence

<220>
<223> Description of Artificial Sequence: Staphylococcus
      hominis Primer

<400> 20
gtaattcgtt accatgttct aa                                                22


<210> 21
<211> 19
<212> DNA
<213> Artificial Sequence

<220>
<223> Description of Artificial Sequence: Escherichia
      coli starting probe

<400> 21
attaacttta ctcccttcc                                                    19


<210> 22

<211> 18
<212> DNA
<213> Artificial Sequence

<220>
<223> Description of Artificial Sequence: Bacteroides
      distasonis starting probe

<400> 22
gacacgtccc gcactttn                                                    18


<210> 23
<211> 19
<212> DNA
<213> Artificial Sequence

<220>
<223> Description of Artificial Sequence:
      Bifidobacterium longum  starting probe

<400> 23
cgcttgctcc ccgantaaa                                                   19


<210> 24
<211> 18
<212> DNA
<213> Artificial Sequence

<220>
<223> Description of Artificial Sequence: Universal
      starting probe

<400> 24
gtattaccgc ggctgctg                                                    18


<210> 25
<211> 21
<212> DNA
<213> Artificial Sequence

<220>
<223> Description of Artificial Sequence: Starting probe

<400> 25
ctctgaaaac ttccgtggat g                                                21

<210> 26
<211> 28
<212> DNA
<213> Artificial Sequence

<220>
<223> Description of Artificial Sequence: Blocking probe

<400> 26
gtatctaatc ctgtttgctc cccacgct 28